# EUROPÄISCHE PATENTSCHRIFT

(11) **EP 1 792 588 B1**
(45) Veröffentlichungstag und Bekanntmachung des Hinweises auf die Patenterteilung: **18.12.2013**
(21) Anmeldenummer: 07003476.4
(22) Anmeldetag: 14.09.1999
(51) Int. Cl.: A61F 2/44, A61F 2/46

(54) **Einsetzinstrument für ein Zwischenwirbelimplantat**
Insert instrument for an implant between vertebrae
Instrument de mise en place pour un implant entre des vertèbres

(43) Veröffentlichungstag der Anmeldung: 06.06.2007
(62) Teilanmeldung aus: 99974009.5
(73) Patentinhaber: Spine Solutions Inc., West Chester, PA 19380 (US)
(72) Erfinder: Beyersdorff, Boris, 10119 Berlin (DE); Marnay, Thierry, 34080 Montpellier (FR)
(74) Vertreter: Erb, Henning

(56) Entgegenhaltungen:
- WO-A-91/13598
- WO-A-98/34552
- FR-A1- 2 737 656

## Beschreibung

Die Erfindung betrifft ein Einsetzinstrument für ein Zwischenwirbelimplantat.

Ein solches Einsetzinstrument ist beispielsweise aus der EP 0 471 821 B1 bekannt. Das Einsetzinstrument ist zangenartig ausgebildet und kann auch dazu dienen, nach dem Einsetzen von Oberteil und Unterteil des Zwischenwirbelimplantats die beiden Wirbelkörper voneinander zu entfernen, um dadurch Raum zur Einführung des Gelenkelements zu erhalten. Dieses Gelenkelement muß bei dem bekannten Instrument mit anderen Instrumenten in den Zwischenraum zwischen Oberteil und Unterteil des Zwischenwirbelimplantats eingeführt werden. Dies ist ein schwieriger Vorgang, bei dem die Gefahr besteht, dass das Gelenkteil gegenüber den beiden anderen Implantatteilen verkantet eingeführt und dadurch beschädigt wird.

Ein ähnlicher Stand der Technik ist aus der FR 2 737 656 Al bekannt, während die WO 98/34552 A1 ein anderes Instrument mit einem anders konzipierten Implantat zeigt.

Es ist auch bekannt, zum Einsetzen von vollständigen Zwischenwirbelimplantaten dies in einer Längsführung bis an die Implantatstelle heranzuführen und dann aus der Führung in den Zwischenwirbelraum abzugeben (DE 43 28 690). Ein solches Instrument ist nur für das Einsetzen vollständiger Zwischenwirbelimplantate geeignet. Außerdem ergibt sich das Problem einer genauen Justierung dieser Führung relativ zu dem Zwischenwirbelraum. Bei Fehljustierungen kann es zu verkantetem Einschub des Zwischenwirbelimplantats kommen, und dies kann zu Verletzungen führen.

Es ist Aufgabe der Erfindung, ein gattungsgemäßes Einsetzinstrument so auszubilden, dass die Einführung des Gelenkelements vereinfacht wird.

Diese Aufgabe wird durch ein Einsetzinstrument der im Anspruch 1 beschriebenen Art erfindungsgemäß gelöst.

Weitere bevorzugte Ausführungsformen sind in den Unteransprüchen angegeben.

Durch die erfindungsgemäße Lösung weist das Einsetzinstrument eine Halteeinrichtung mit einer lösbaren Verriegelung auf. Durch diese lösbare Verriegelung wird das an dem Arm gehaltene Implantatteil unlösbar mit dem Arm verbunden. Erst nach Entriegelung dieser Verriegelung ist es möglich, Implantatteil und Einsetzinstrument wieder zu trennen.

Dadurch wird eine unbeabsichtigte Trennung des Einsetzinstruments von den Implantatteilen vermieden. Es ist sogar möglich, auf diese Weise bereits implantierte Implantatteile wieder aus dem Zwischenwirbelraum herauszuziehen, falls dies notwendig werden sollte.

Die Verriegelung erfolgt durch eine Verdrehung eines Riegels um eine Drehachse, die parallel zu Längsachse des Armes verläuft, an dem die Halteeinrichtung angeordnet ist.

Insbesondere ist vorgesehen, daß der die Halteeinrichtung tragende Arm oder ein Teil desselben um seine Längsachse verdrehbar ist und einen Riegel trägt, der in einer Stellung das an der Halteeinrichtung gehaltene Teils des Zwischenwirbelimplantats unlösbar mit diesem verriegelt und in einer anderen Stellung freigibt.

Die Halteeinrichtung ist ein in eine Aufnahmebohrung am gehaltenen Teil des Zwischenwirbelimplantats eingreifender Stift und der Riegel ist ein seitlich von diesem abstehender Vorsprung, der in einer Winkelstellung des Stifts in einen entsprechenden Rücksprung des Implantatteils eingreift, in einer anderen Winkelstellung jedoch aus diesem Rücksprung austritt.

Man erhält auch ein kombiniertes Einsetzinstrument, welches zunächst der Handhabung von Oberteil und Unterteil des Implantats dient und mit dem Oberteil und Unterteil in der gewünschten Position in den Zwischenwirbelraum einbringbar sind. Durch die verschwenkbare Lagerung der Arme können Oberteil und Unterteil dann unter Aufweitung des Zwischenwirbelraums in an sich bekannter Weise voneinander entfernt werden, so daß ein Einführraum für das Gelenkelement zwischen ihnen entsteht. In diesen Einführraum wird das Gelenkelement dann über die Führung in einem der beiden Arme des Einsetzinstruments direkt eingeschoben, wobei durch die Verbindung der beiden Arme des Einsetzinstruments mit den in den Zwischenwirbelraum eingesetzten Teilen des Implantats eine zuverlässige Justierung der Längsführung für das Gelenkelement gewährleistet ist. Außerdem ist sichergestellt, daß das Gelenkelement exakt in der gewünschten Relativposition zu den anderen beiden Implantatteilen in den Zwischenwirbelraum eingeführt wird.

Sowohl das Einsetzen von Oberteil und Unterteil des Implantats als auch des Einführen des Gelenkelements kann somit mit einem einzigen Instrument erfolgen, ein Absetzen und Auswechseln ist nicht mehr notwendig. Dieses Einsetzinstrument übernimmt eine größere Anzahl von Funktionen, nämlich die des Einsetzens von Oberteil und Unterteil des Zwischenwirbelimplantats, die der Aufweitung des Zwischenwirbelraums und schließlich die der Einführung des Gelenkelements in den Zwischenraum zwischen Oberteil und Unterteil des Implantats.

Günstig ist es, wenn die Längsführung durch in Längsnuten eingreifende Vorsprünge gebildet wird.

Beispielsweise kann vorgesehen sein, daß in einem der Arme in einem in dessen Längsrichtung verlaufendem Aufnahmeraum für das Gelenkelement einander gegenüberliegende Nuten angeordnet sind, in die das Gelenkelement mit seitlichen Vorsprüngen eingreift.

Bei einer besonders bevorzugten Ausführungsform ist vorgesehen, daß der die Längsführung umfassende Arm zwei im Abstand und parallel zueinander angeordnetem stabförmige Schenkel aufweist, die zwischen sich einen Aufnahmeraum für das Gelenkelement bilden und dieses längs des Aufnahmeraums zwischen sich führen.

Günstig ist es, wenn die Längsführung an ihrem der Schwenklagerung der Arme benachbarten Ende einen Einsetzbereich bildet, an dem das Gelenkelement in die Längsführung einschiebbar ist. Dieser Einsetzbereich kann beispielsweise dadurch erfolgen, daß Längsnuten stirnseitig offen sind. In einem anderen Ausführungsbeispiel kann vorgesehen sein, daß die Längsführung erst in einem Abstand von der Schwenklagerung beginnt, der der Länge des einzusetzenden Gelenkelements entspricht.

Bei einer besonders bevorzugten Ausführungsform ist vorgesehen, daß die Längsführung des einen Armes in eine Längsführung des an dem Arm gehaltenen Teils des Zwischenwirbelimplantats übergeht. Man erhält dadurch eine kontinuierliche Längsführung für das Gelenkelement einmal längs des Armes und zum anderen auch längs des einen Teils des Zwischenwirbelimplantats, so daß eine absolut präzise Einführung des Gelenkelements in das angeschlossene Teil des Zwischenwirbelimplantats gewährleistet ist. Dieses an den Arm angeschlossene Teil des Implantats bildet während des Einschubvorgangs praktisch ein Teil des Einsetzinstrument, das nach dem Einführen des Gelenkelements vom Einsetzinstrument abgetrennt wird und als Implantatteil im Zwischenwirbelraum verbleibt.

Bei einer weiteren bevorzugten Ausführungsform ist vorgesehen, daß das Einsetzinstrument einen in die Längsführung einsetzbaren Vorschubkörper umfaßt, der mit einem stabförmigen Schubelement verbunden ist. Damit kann das Gelenkelement der Längsführung bis in den Zwischenwirbelraum vorgeschoben werden.

Es ist besonders vorteilhaft, wenn die beiden Arme gemäß einer bevorzugten Ausführungsform der Erfindung an ihren freien Enden derart nebeneinander angeordnet sind, daß die Halteeinrichtungen sich in Richtung der Schwenkbewegung der Arme zumindest teilweise überlappen. Man kann dadurch eine sehr geringe Bauhöhe des Einsetzinstruments realisieren. Diese Bauhöhe liegt in der Größenordnung der Spaltbreite des Zwischenwirbelraums und es ist außerdem möglich, dadurch die beiden Teile des Implantats, die mit den Armen des Einsetzinstruments verbunden werden, ebenfalls sehr dicht aneinander zu führen und dadurch eine sehr geringe Bauhöhe zu realisieren. Auf diese Weise können diese beiden Teile des Implantats ohne große Aufweitung des Zwischenwirbelraums in diesen eingeführt werden. Die Aufweitung des Zwischenwirbelraums erfolgt erst nach der Einführung dieser Teile des Zwischenwirbelimplantats durch Auseinanderschwenken der diese beiden Teile des Implantats haltenden Arme.

Es ist vorteilhaft, wenn die Schwenklagerungen der beiden Arme einen Abstand voneinander haben, so daß die Arme in einer Einsetzstellung des Oberteils und des Unterteils des Zwischenwirbelimplantats, bei der die freien Enden der Arme einander maximal angenähert sind, am gelagerten Ende einen größeren Abstand voneinander haben als am freien Ende. Auch dies trägt dazu bei, die Bauhöhe des Einsetzinstruments und der daran gehaltenen Implantatteile beim Einsetzen so gering wie möglich zu gestalten.

Außerdem ist es bei dieser Anordnung gemäß einer bevorzugten Ausführungsform möglich, daß ein Spreizelement vorgesehen ist, das sich an beiden Armen abstützt und längs der Arme in Richtung auf das freie Ende der Arme vorschiebbar ist und dabei die Arme auseinander schwenkt. Damit ist allein durch Vorschieben des Spreizelements längs der Arme das Aufweiten des Zwischenwirbelraums möglich, nachdem Oberteil und Unterteil des Zwischenwirbelimplantats in den Zwischenwirbelraum eingesetzt sind.

Dabei ist es günstig, wenn mindestens einer der beiden Arme eine Längsführung für den Spreizkörper aufweist, so daß dieser längs der Arme definiert geführt wird.

Außerdem kann am Spreizkörper eine Vorschubstange angeordnet sein, mit dessen Hilfe der Spreizkörper längs der Arme verschoben wird.

Bei einer besonders bevorzugten Ausführungsform ist dabei vorgesehen, daß die Vorschubstange als Zahnstange ausgebildet ist, die mit einem Antriebszahnrad im Bereich der Schwenklagerung der Arme kämmt. Dadurch ist eine sehr gefühlvolle Vorschubbewegung des Spreizkörpers längs der Arme möglich, wobei auch große Kräfte über die Zahnverbindung übertragen werden können.

Die Haltevorrichtungen, mit denen die Implantatteile an den Armen gehalten sind, können sehr unterschiedlich ausgebildet sein, besonders bevorzugt wird eine Ausgestaltung, bei der die Haltevorrichtungen Stifte sind, die in Öffnungen des Oberteiles beziehungsweise des Unterteils des Zwischenwirbelimplantats eingreifen.

Bei einer bevorzugten Ausführungsform ist vorgesehen, daß die Haltevorrichtungen zumindest an einem der Arme um eine Schwenkachse verschwenkbar sind, die im Bereich des freien Endes des Armes angeordnet ist und parallel zur Schwenkachse des Armes verläuft, und daß die Haltevorrichtungen nach dem Verschwenken um diese Schwenkachse in unterschiedlichen Winkelstellungen feststellbar sind. Dadurch ist es möglich, die Neigung der bei den Implantatteile relativ zu einander geringfügig zu verändern, beispielsweise im Bereich von 1° bis 5°, so daß neben der Implantathöhe auch der Implantatwinkel so gewählt werden kann, wie es der korrekten Positionierung der Wirbelkörper entspricht.

Bei einer bevorzugten Ausführungsform kann dabei zur Feststellung der Winkelstellung ein Fixierstift vorgesehen sein, der in bei unterschiedlicher Winkelstellung miteinander ausgerichtete Bohrungen einschiebbar ist.

Bei einer besonders bevorzugten Ausführungsform ist vorgesehen, daß der die Längsführung aufweisende Arm zwei parallel zueinander angeordnete Schenkel aufweist, die zwischen sich einen Aufnahmeraum für das Gelenkelement ausbilden, und daß der andere Arm mittig zwischen diesen Schenkeln verläuft, so daß er mit seinem freien Ende zwischen die Schenkel eintauchen kann.

Weiterhin kann vorgesehen sein, daß ein zwischen den Armen angeordnetes, längs derselben verschiebliches Spreizelement auf der Oberseite der beiden Schenkel aufliegt und mit einem Vorsprung zwischen die beiden Schenkel in den Aufnahmeraum eingreift. Dadurch ergibt sich eine Führung des Spreizelements längs der Arme.

Zusätzlich kann das Spreizelement an seiner Oberseite eine Vertiefung aufweisen, in die der andere Arm eintaucht. Auch diese trägt zur Führung des Spreizelements bei.

Die Schenkel des einen Armes können im Querschnitt rechteckig sein, der andere Arm kann im Querschnitt kreisförmig sein.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht des Einsetzinstruments nach dem Einführen des Oberteils und des Unterteils eines Zwischenwirbelimplantats in den Zwischenwirbelraum, vor dem Aufspreizen des Zwischenwirbelraums und vor dem Einführen des Gelenkelements in den Zwischenwirbelraum;

- Figur 2:: eine Draufsicht auf den oberen Arm des Einsetzinstruments der Figur 1 mit an ihm gehaltenem Oberteil des Zwischenwirbelimplantats;
- Figur 3:: eine Seitenansicht in Richtung des Pfeiles A in Figur 2;
- Figur 4:: eine Seitenansicht des unteren Armes mit daran gehaltenem Unterteil des Zwischenwirbelimplantats, welches längs der Linie 4-4 in Figur 5 geschnitten dargestellt ist;
- Figur 5:: eine Draufsicht auf den unteren Arm in Richtung des Pfeiles B in Figur 4;
- Figur 6:: eine perspektivische Ansicht des Einsetzinstruments mit daran gehaltenem Oberteil und Unterteil in der Einsetzposition mit maximal angenäherten Implantatteilen;
- Figur 7:: eine perspektivische Ansicht des Einsetzinstruments der Figur 6 nach dem Einsetzen des Oberteils und des Unterteils des Zwischenwirbelimplantats in den Zwischenwirbelraum und nach dem Aufweiten desselben kurz vor dem Einschieben des Gelenkelements zwischen Oberteil und Unterteil des Zwischenwirbelimplantats;
- Figur 8:: eine Seitenansicht des vorderen Teils des Einsetzinstruments der Figur 7 kurz vor dem Einschieben des Gelenkelements zwischen Oberteil und Unterteil des Zwischenwirbelimplantats;
- Figur 9:: eine Schnittansicht längs der Linie 9-9 in Figur 8 und

- Figur 10:: eine Ansicht ähnlich Figur 8 nach dem Einschieben des Gelenkelements zwischen Oberteil und Unterteil des Zwischenwirbelimplantats.

Das in der Zeichnung dargestellte Einsetzinstrument 1 dient dem Einsetzen eines Zwischenwirbelimplantats 2 in den von zwei Wirbelkörpern 3, 4 begrenzten Zwischenwirbelraum 5.

Das Zwischenwirbelimplantat 2 umfaßt dabei ein im wesentlichen plattenförmiges Oberteil 6 mit einer oberen ebenen Anlagefläche 7 und von dieser abstehenden Verankerungselementen 8 sowie ein ebenfalls plattenförmiges Unterteil 9 mit einer ebenen äußeren Anlagefläche 10 und von dieser abstehenden Verankerungselementen 11.

Das Oberteil 6 weist an seiner dem Unterteil 9 zugewandten Seite eine kugelkalottenförmige Lagerfläche 12 auf. In das Unterteil 9 ist eine Vertiefung 13 eingearbeitet, die zu einer Seite hin offen ist und die einen Einschubraum für ein ebenfalls Teil des Zwischenwirbelimplantats 2 bildendes Gelenkelement 14 bildet. Dieses Gelenkelement 14 weist einen plattenförmigen, im wesentlichen rechteckigen Sockel 15 und einen einseitig von diesem zentral abstehenden Lagervorsprung 16 auf, dessen Oberseite eine kugelkalottenförmige Lagerfläche 17 ausbildet.

Das Gelenkelement 14 kann von der offenen Seite in die Vertiefung 13 eingeschoben werden, wobei die seitlichen Ränder des Sockels 15 in seitliche Nuten 18 im Unterteil 9 eingreifen, so daß das Gelenkelement 14 längs dieser Nuten 18 geführt in die Vertiefung 13 einschiebbar ist.

Im implantierten Zustand greift die Lagerfläche 17 in die konkave Lagerfläche 12 des Oberteils ein, so daß Oberteil 6 und Unterteil 9 sich über das Gelenkelement gegeneinander abstützen und gegeneinander verschwenkbar sind.

Sowohl das Oberteil 6 als auch das Unterteil 9 weisen an einer Seitenfläche im wesentlichen parallel zu den Anlageflächen 7 beziehungsweise 10 verlaufende Einstecköffnungen 19 auf, in die Haltestifte 20 des Einsetzinstruments 1 einsteckbar sind.

Dieses Einsetzinstrument 1 weist einen ersten länglichen Arm 21 mit zwei im Abstand zueinander und parallel zueinander verlaufenden Schenkeln 22, 23 auf, die einseitig um ihre Längsachse verdrehbar an einem Lagerbock 24 gehalten sind. Beide Schenkel 22 und 23 weisen einen quadratischen Querschnitt auf und bilden stangenförmige, lange Elemente, die an ihrem freien Ende in Verlängerung der Drehachse der Schenkel jeweils einen der Haltestifte 20 tragen.

An diesen Haltestiften 20 der Schenkel 22 und 23 sind außerdem radial abstehende Riegelvorsprünge 25 vorgesehen, die beispielsweise durch in die Haltestifte 20 radial eingesteckte Stifte realisiert werden können, die in einer Winkelstellung der Schenkel 22 in seitliche Rücksprünge 26 des Unterteils 9 eingreifen. Diese Rücksprünge 26 sind zum Oberteil 6 hin offen, so daß durch Verdrehung der Schenkel 22 und 23 um 90° die Riegelvorsprünge 25 so verdreht werden können, daß sie aus den Rücksprüngen 26 austreten. Solange die Riegelvorsprünge 25 in die Rücksprünge 26 eingreifen, sind die Schenkel 22 und 23 bei in die Einstecköffnungen 19 eingesteckten Haltestiften 20 lösbar mit dem Unterteil 9 verbunden. Werden die Riegelvorsprünge 25 jedoch durch Verdrehung der Schenkel 22 und 23 aus den Rücksprüngen 26 herausgedreht, können die Haltestifte 20 aus den Einstecköffnungen 19 herausgezogen werden, so daß eine Verschiebung der Schenkel 22 und 23 gegenüber dem Unterteil 9 möglich wird und damit ein Einschieben oder Trennen.

Die Schenkel 22 und 23 können durch eine in der Zeichnung nicht dargestellte Rastung in den Endstellungen lösbar festgelegt werden, in denen der Riegelvorsprung 25 in den Rücksprung 26 eingreift beziehungsweise in denen er vollständig aus dem Rücksprung 26 austritt.

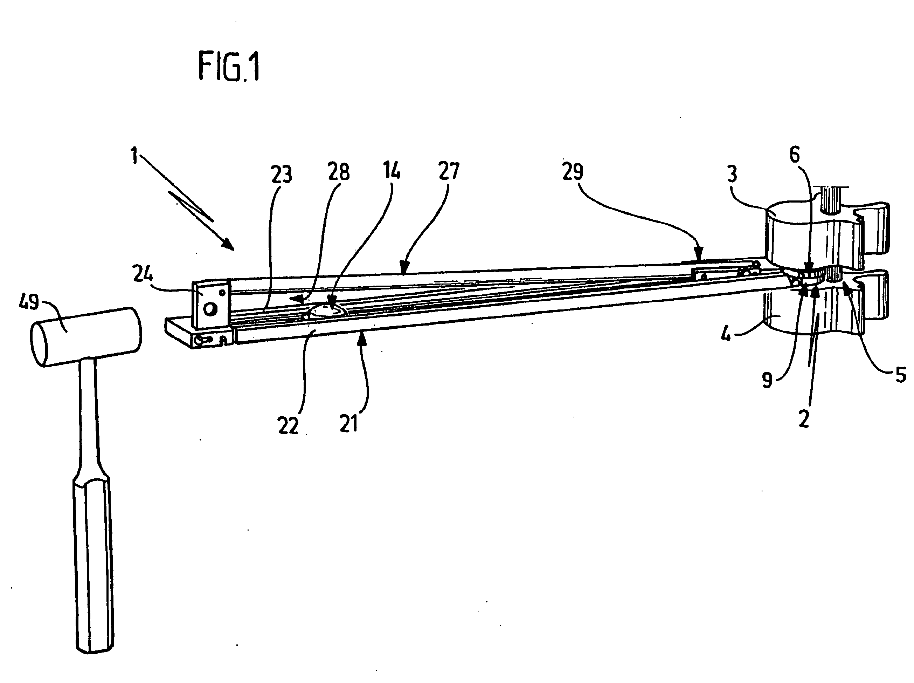

An dem Lagerbock 24 ist im Abstand von der Ebene, die durch die beiden Schenkel 22 und 23 aufgespannt wird, ein zweiter Arm 27 um eine Drehachse verschwenkbar gelagert, die quer zur Längsrichtung der Schenkel 22 und 23 und parallel zu der von diesen aufgespannten Ebene verläuft. Der Arm 27 ist dabei etwa in der Mitte zwischen den beiden Schenkeln 22 und 23 angeordnet, so daß das freie Ende des Arms 27 in den Zwischenraum 28 zwischen den beiden Schenkeln 22 und 23 eintreten kann. Aufgrund des Abstandes der Lagerstelle der Armes 27 von der durch die Schenkel 22 und 23 aufgespannten Ebene nimmt dabei der Abstand der Armes 27 vom Arm 21 kontinuierlich ab, wie dies aus der Darstellung der Figur 1 deutlich wird.

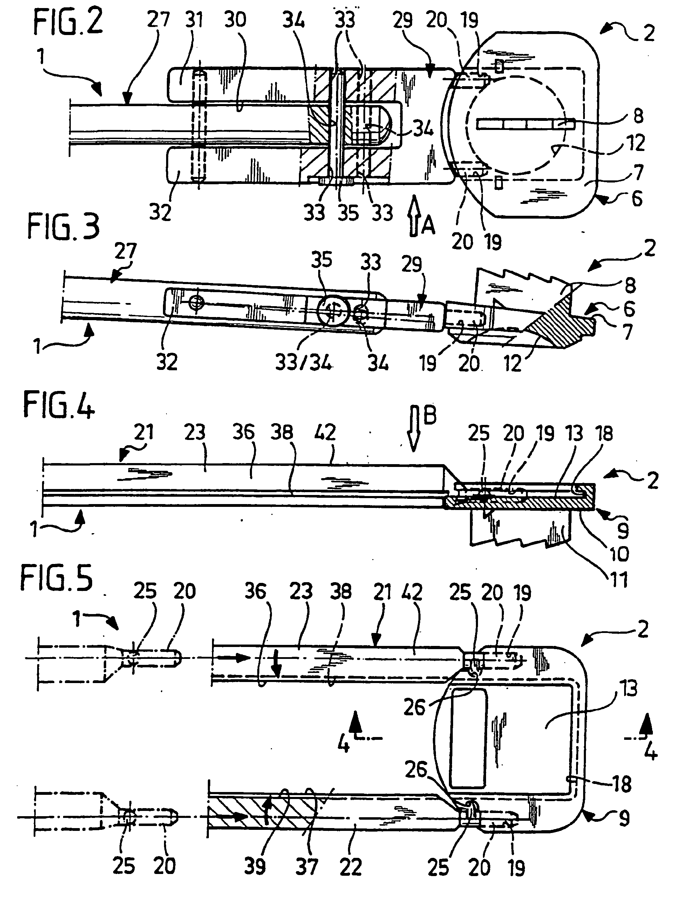

Der Arm 27 ist im Querschnitt kreisförmig ausgebildet und trägt an seinem freien Ende einen U-förmigen Halter 29, der das freie Ende des Armes 27 im Zwischenraum 30 zwischen zwei parallel verlaufenden Schenkeln 31, 32 aufnimmt. Im Bereich der freien Ende der Schenkel 31, 32 sind der Halter 29 und der Arm 27 um eine parallel zur Schwenkachse des Armes 27 verlaufende Drehachse verschwenkbar miteinander verbunden. Dadurch kann der Halter 29 relativ zum Arm 27 unterschiedliche Winkelstellungen einnehmen. In Figur 3 sind zwei sich um einen kleinen Winkelbetrag unterscheidende Winkelstellungen mit strichpunktierten Linien eingetragen. Zur Festlegung des Halters 29 in unterschiedlichen Winkelstellungen sind sowohl in den Schenkeln 31 und 32 als auch im Arm 27 Querbohrungen 33 beziehungsweise 34 vorgesehen, und zwar in Längsrichtung versetzt mehrere derartige Paare von Querbohrungen, die bei unterschiedlichen Stellungen des Halters 29 relativ zum Arm 27 miteinander ausgerichtet sind. In diese Paare von Querbohrungen 33, 34 kann ein Fixierstift 35 eingeschoben werden. Da die zusammengehörenden Querbohrungen 33, 34 bei den verschiedenen Paaren eine unterschiedliche Position einnehmen, ergibt sich für jedes Paar von Querbohrungen beim Einsetzen eines Fixierstiftes 35 eine unterschiedliche Winkellage relativ zum Arm 27. Die Schwenkwinkel liegen dabei in der Größenordnung einiger Grad, beispielsweise wird insgesamt ein Bereich überstrichen, der zwischen 1° und 5° liegen kann.

An dem Halter 29 sind Haltestifte 20 angeordnet, die in der beschriebenen Weise in Einstecköffnungen 19 des Oberteils 6 einschiebbar sind. Durch die unterschiedliche Winkelstellung des Halters 29 ist es möglich, das Oberteil 6 gegenüber dem Unterteil 9, das an den Schenkeln 22 und 23 gehalten ist, in geringem Umfang zu verkippen.

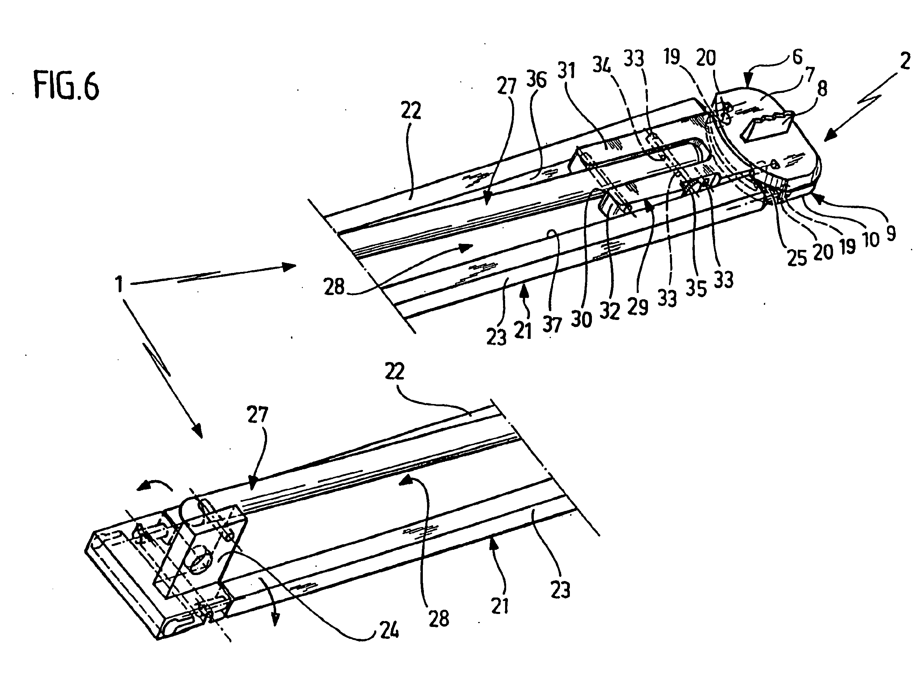

Die Breite des Halters 29 ist so gewählt, daß der Halter 29 in den Zwischenraum 28 zwischen den beiden Schenkeln 22 und 23 hineinpaßt, so daß die Haltestifte 20 am Halter 29 und an den Schenkeln 22 und 23 praktisch nebeneinander angeordnet werden können. Dadurch ist es möglich, Oberteil 6 und Unterteil 9 in einer maximal angenäherten Position an den beiden Armen 21 und 27 zu halten. Diese Position wird als Einsetzposition bezeichnet (Figuren 1 und 6).

Die beiden Schenkel 22 und 23 tragen in den Innenflächen 36, 37, die einander zugewandt sind, wenn die Riegelvorsprünge 25 in die Rücksprünge 26 eingreifen, einander gegenüberliegende Längsnuten 38, 39, die eine Längsführung für das Gelenkelement 14 ausbilden. Die Dimensionierung dieser Längsnuten 38 und 39 entspricht der der Seitenränder des Sockels 15 des Gelenkelements 14, so daß das Gelenkelement 14 in dem Zwischenraum 28 zwischen den Schenkeln 22 und 23 in Längsrichtung geführt wird, wenn die Seitenränder des Sockels 15 in die Längsnuten 38 und 39 eintauchen. Diese Längsnuten 38 und 39 enden in einem Abstand vor dem Lagerbock 24, der ein Einsetzen des Sockels 15 in die Längsnuten 38, 39 ermöglicht, und diese Längsnuten 38 und 39 setzen sich bis an das freie Ende der Schenkel 22 und 23 fort. Dort gehen sie unmittelbar über in die beidseitig der Vertiefung 13 angeordneten Nuten 18, die der Aufnahme des Sockels 15 dienen. Man erhält damit eine von den Schenkeln 22 und 23 direkt bis in das Unterteil 9 des Zwischenwirbelimplantats 2 führende Führungsbahn für das Gelenkelement 14.

In die Längsnuten 38 und 39 ist außerdem ein plattenförmiger Vorschubkörper 40 einsetzbar, der gelenkig mit einer Schubstange 41 verbunden ist. Mittels dieser Schubstange 41 läßt sich das in die Längsnuten 38 und 39 eingesetzte Gelenkelement 14 längs seiner Führungsbahn vorschieben. Der Vorschubkörper 40 wird dazu nach dem Gelenkelement 14 in die durch die Längsnuten 38 und 39 gebildete Führungsbahn eingeführt.

Auf der ebenen Oberseite 42 der Schenkel 22 und 23 stützt sich ein den Zwischenraum 28 zwischen den beiden Schenkeln 22 und 23 überbrückendes Spreizelement 43 ab, welches mit einem Vorsprung 44 in den Zwischenraum 28 geringfügig eintaucht und dadurch quer zur Längsrichtung der Schenkel 22 und 23 geführt wird. Dieses Spreizelement 43 weist an seinem den Schenkeln 22 und 23 abgewandten Ende eine Vertiefung 45 mit kreisbogenförmigem Querschnitt auf, in die der Arm 27 eintaucht. Das Spreizelement 23 ist mit einer als Zahnstange ausgebildeten Schubstange 46 verbunden. Diese kämmt mit einem Zahnrad 47, welches am Lagerbock 24 verdrehbar gelagert ist und mittels eines Griffteils 48 verdreht werden kann. Bei dieser Verdrehung wird die Schubstange 46 verschoben, und dies führt zu einer Längsverschiebung des Spreizelements 43 längs der Schenkel 22 und 23. Beim Vorschieben des Spreizelements 43 wird dadurch der Arm 27 von den Schenkeln 22 und 23 weggeschwenkt. Die Arme 27 und 21 werden also gespreizt, so daß dadurch das Oberteil 6 und das Unterteil 9 voneinander entfernt werden. Dies führt auch zu einem Auseinanderdrücken der Wirbelkörper 3 und 4 und damit zu einer Aufweitung des Zwischenwirbelraums 5.

Das beschriebene Einsetzinstrument besteht vorzugsweise aus einem körperverträglichen Metall, beispielsweise aus Titan oder einer Titanlegierung. Dasselbe gilt hinsichtlich des Oberteils 6 und des Unterteils 9 des Zwischenwirbelimplantats 2. Das Gelenkelement 14 wird aus einem körperverträglichen Kunststoffmaterial hergestellt, beispielsweise aus Polyethylen, und auch das Spreizelement 43 besteht vorzugsweise aus einem Kunststoffmaterial, um ein gutes Gleiten gegenüber den Schenkeln 22 und 23 und gegenüber dem Arm 27 zu gewährleisten.

Zum Einsetzen des Zwischenwirbelimplantats 2 in einen Zwischenwirbelraum 5 wird zunächst nach Entfernung der Bandscheibe aus dem Zwischenwirbelraum 5 dieser in geeigneter Weise vorbereitet, beispielsweise können senkrechte Nuten in die Wirbelkörper 3, 4 eingeschlagen werden, die die Verankerungselemente 8 beziehungsweise 11 des Zwischenwirbelimplantats 2 aufnehmen.

Nach entsprechender Vorbereitung werden das Oberteil 6 und das Unterteil 9 auf die Arme 27 beziehungsweise 21 aufgesteckt. Das Unterteil 9 wird durch Verdrehung der Schenkel 22, 23 mit dem Arm 21 verriegelt, wobei die Riegelvorsprünge 25 in die Rücksprünge 26 des Unterteils 9 eingreifen, und die beiden Arme 21 und 27 werden in die Einsetzposition verschwenkt, bei der das Oberteil 6 und das Unterteil 9 einander maximal angenähert sind. Diese beiden Teile haben also eine sehr geringe Bauhöhe. In dieser Einsetzposition werden Oberteil 6 und Unterteil 9 in den vorbereiteten Zwischenwirbelraum 5 eingeführt, beispielsweise durch Einschlagen mittels eines hammerähnlichen Instruments 49 (Figur 1). Dabei kann die Neigung, die das Oberteil 6 gegenüber dem Unterteil 9 einnimmt, durch Verschwenken des Halters 29 gegenüber dem Arm 27 vorgewählt werden. In der gewünschten Position wird die Winkelstellung durch den Fixierstift 35 fixiert.

Nach diesem Einsetzen von Oberteil 6 und Unterteil 9 werden nacheinander das Gelenkelement 14 und der Vorschubkörper 40 in die durch die Längsnuten 38, 39 gebildete Führungsbahn eingeschoben. Außerdem werden der Vorschubkörper 40 sowie die Schubstange 41 und das Zahnrand 47 in das Instrument eingesetzt.

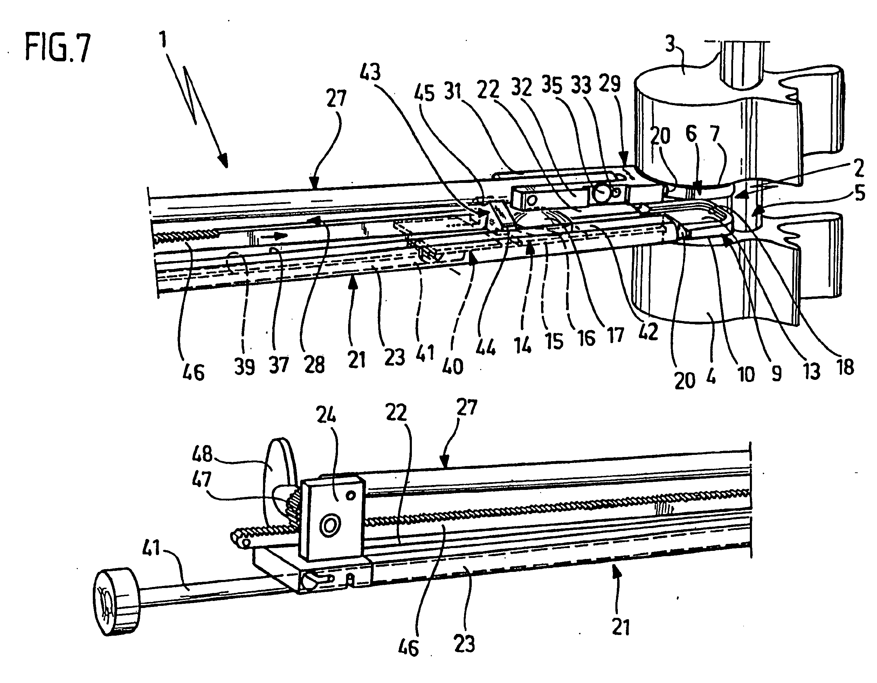

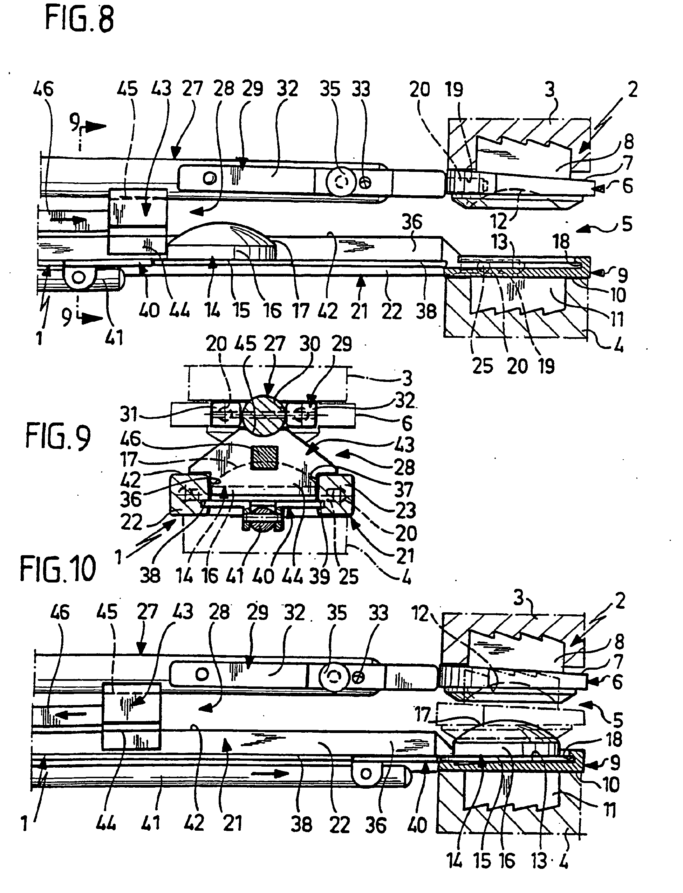

Durch Verdrehen des Zahnrads 47 und Vorschieben des Spreizelements 43 werden die Arme 21 und 27 auseinandergespreizt. Dies führt zu einer Vergrößerung des gegenseitigen Abstandes von Oberteil 6 und Unterteil 9 und damit zu einer Aufweitung des Zwischenwirbelraums 5 (Figuren 7 bis 9). Die Aufweitung wird dabei so stark gewählt, daß mittels des Vorschubkörpers 40 das Gelenkelement 14 in die Vertiefung 13 des Unterteils 9 eingeschoben werden kann (Figur 10). Daran anschließend wird durch Zurückziehen des Spreizelements 43 der Abstand zwischen Oberteil 6 und Unterteil 9 wieder verringert, bis die Lagerflächen 12 und 17 ineinander eingreifen und die Teile des Zwischenwirbelimplantats 2 damit ihre Endposition erreicht haben (Figur 10, strichpunktierte Darstellung des Oberteils 6).

Durch Verdrehung der Schenkel 22 und 23 um ihre Längsachse wird der Eingriff des Riegelvorsprungs 25 in den Rücksprung 26 aufgehoben, und dann kann das Einsetzinstrument 1 von dem ordnungsgemäß eingesetzten Zwischenwirbelimplantat 2 abgezogen werden.

## Patentansprüche

1. Einsetzinstrument für ein Zwischenwirbelimplantat (2) mit einem Oberteil (6) und einem Unterteil (9), die an benachbarten Wirbelkörperflächen eines Zwischenwirbelraumes anliegen und miteinander in Anlage und relativ beweglich zueinander sind, wobei das Oberteil (6) und das Unterteil (9) an ihren Seiten jeweils ein Paar Öffnungen (19) aufweisen, die seitliche Rücksprünge (26) haben und in deren Verlaufsrichtung das Instrument (1) an dem Implantat (2) ansetzbar ist, das Instrument (1) ein Paar Armelemente (21, 27) aufweist, die schwenkbar gegeneinander gelagert sind und jeweils ein Paar Haltevorrichtungen in der Form von Stiften (20) aufweisen, **dadurch gekennzeichnet, dass** die Stifte (20) radial abstehende Riegelvorsprünge (25) aufweisen und derart angeordnet sind, dass sie jeweils in zwei der vier Öffnungen (19) des Implantats (2) steckbar sind, um das Instrument (1) mit dem Implantat (2) zu verriegeln, und die Armelemente (21, 27) derart beweglich ausgebildet sind, dass sie, nachdem die Stifte (20) in die Öffnungen (19) eingerückt sind, verdrehbar sind, bis die Riegelvorsprünge (25) der Stifte (20) in den Rücksprüngen (26) eingreifen, um die Stifte (20) in den Öffnungen (19) und dadurch das Instrument (1) mit dem Implantat (2) zu verriegeln.

2. Einsetzinstrument nach Anspruch 1, **dadurch gekennzeichnet, dass** wenigstens ein Drehelement vorgesehen ist, das das Verdrehen des Armelements (21) auslöst, um die Stifte (20) in den Öffnungen (19) festzulegen.

3. Einsetzinstrument nach Anspruch 1, **dadurch gekennzeichnet, dass** die Armelemente ein oberes Armelement (27), das in die Öffnungen (19) in dem Oberteil (6) eingreift, und ein unteres Armelement (21) aufweist, das in die Öffnungen des Unterteils (9) eingreift.

4. Einsetzinstrument nach Anspruch 3, **dadurch gekennzeichnet, dass** wenigstens einer der Stifte (20) um seine Achse verdrehbar ist, um ihn gegen das Implantat (2) zu verriegeln.

5. Einsetzinstrument nach Anspruch 4, **dadurch gekennzeichnet, dass** wenigstens ein drehbares Element (22, 23) in wenigstens einem Armelement (21) vorgesehen ist, um den jeweiligen Stift (20) zu drehen und das Implantat (2) mit dem Instrument (1) zu verriegeln.

6. Einsetzinstrument nach Anspruch 5, **dadurch gekennzeichnet, dass** das untere Armelement zwei Schenkel (22, 23) aufweist, die jeweils ein drehbares Element bilden.

## Claims

1. An insertion instrument for an intervertebral implant (2) of the type having upper (6) and lower (9) parts which engage adjacent vertebral surfaces of an intervertebral space and which are engaged with each other and movable relative to each other and which upper (6) and lower (9) parts have a pair of bores (19) in a side thereof in the path along which the instrument (1) approaches the implant (2), the bores including a lateral recess (26),
said instrument (1) having a pair of arm members (21, 27) that are pivotally coupled to each other, each arm member having a pair of retention members in the form of pins (20), **characterized in that** the pins (20) include protrusions (25) extending radially from the pin, are positioned to engage two of the four apertures (19) of the implant (2) as the instrument moves into engagement with the implant, and
wherein the arm members (21, 27) are moveable such that, after the pins (20) have entered the bores (19) the arm members (21, 27) can be rotated until the protrusion (25) of each of the pins (20) is positioned within the recess (26) thereby securing, the pins in the bores and securing the instrument (1) and the implant (2) together.

2. An instrument according to claim 1, **characterized in that** at least a rotating element is provided which, causes said turning movement of the arm element (21) to secure the pins (20) in the bores (19).

3. An instrument according to claim 1, **characterized in that** the arm members (27) include an upper arm member which engages the bores (19) in the upper part (6) and a lower arm member (21) which engages the bores in the lower part (9).

4. An instrument according to claim 3, **characterized in that** at least one of the pins (20) is rotatable about its axis to secure that pin to the implant (2).

5. An instrument according to claim 4, **characterized in that** it includes at least one rotary element (22, 23) in one of the arm members (21) to rotate its pin (20) to secure the instrument (1) to the implant (2).

6. An instrument according to claim 5, **characterized in that** the lower arm member comprises two legs (22, 23), each of the two legs forming a rotary element.

## Revendications

1. Instrument pour l'insertion d'un implant intervertébral (2) comprenant une partie supérieure (6) et une partie inférieure (9) s'appliquant sur des surfaces voisines de corps de vertèbre d'un espace intervertébral et qui sont en contact l'une avec l'autre et mobile l'une par rapport à l'autre, la partie supérieure (6) et la partie inférieure (9) comportant respectivement latéralement une paire d'ouvertures (19) comportant des retraits latéraux (26) et dans la direction d'extension desquelles l'instrument (1) peut être positionné sur l'implant (2), cet instrument (1) comportant les bras (21, 27) qui sont montés pivotant l'un par rapport à l'autre et comportent respectivement une paire de dispositifs de retenue réalisés sous la forme de broches (20),
**caractérisé en ce que**
les broches (20) comportent des saillies de verrouillage (25) dirigées radialement vers l'extérieur et positionnées de façon à pouvoir être respectivement enfichables dans deux des quatre ouvertures (19) de l'implant (2) pour bloquer l'instrument (1) avec l'implant (2) et les bras (21, 27) sont mobiles de façon à pouvoir être déplacés en rotation après que les broches (20) aient été introduites dans les ouvertures (19) jusqu'à ce que les saillies de verrouillage (25) des broches (20) viennent en prise dans les retraits (26) pour bloquer les broches (20) dans les ouvertures (19) et ainsi l'instrument avec l'implant (2).

2. Instrument conforme à la revendication 1,
**caractérisé en ce qu'**
il est prévu au moins un élément rotatif qui déclenche la rotation du bras (21) pour fixer les broches (20) dans les ouvertures (19).

3. Instrument conforme à la revendication 1,
**caractérisé en ce que**
les bras comportent un bras supérieur (27) qui vient en prise dans les ouvertures (19) situées dans la partie supérieure (6) et un bras inférieur (21) qui vient en prise dans les ouvertures de la partie inférieure (9).

4. Instrument conforme à la revendication 3,
**caractérisé en ce qu'**
au moins l'une des broches (20) est mobile en rotation autour de son axe pour permettre de la bloquer par rapport à l'implant (2).

5. Instrument conforme à la revendication 4,
**caractérisé en ce qu'**
il est prévu au moins un élément mobile en rotation (22, 23) dans au moins un bras (21) pour permettre de faire tourner la broche (20) respective et de bloquer l'implant (2) avec l'instrument (1).

6. Instrument conforme à la revendication 5,
**caractérisé en ce que**
le bras inférieur comporte deux branches (22, 23) qui forment respectivement un élément mobile en rotation.
